# EUROPEAN PATENT APPLICATION

(11) **EP 4 516 363 A1**
(43) Date of publication of application: **05.03.2025**
(21) Application number: 23792109.3
(22) Date of filing: 14.04.2023
(51) Int. Cl.: A62B 18/00, A62B 18/08, A62B 23/02, A61L 9/20, G06F 3/16, G10L 17/24, H01F 7/02

(54) **NON-ENCLOSING DEVICE FOR BLOCKING VIRUSES AND BACTERIA**

(30) Priority: 23.04.2022 KR 20220050471
(71) Applicant: Yun, Sung Eun, Goyang-si, Gyeonggi-do 10411 (KR)
(72) Inventor: Yun, Sung Eun, Goyang-si, Gyeonggi-do 10411 (KR)
(74) Representative: Hautier IP - MC/EP
(86) International application number: PCT/KR2023/005081
(87) International publication number: WO 2023/204532

(57) **Abstract**

A non-enclosing device for blocking viruses and bacteria, according to the present invention, comprises: a main body that can be attached/detached to/from the body of a user; and a blocking part provided at the main body so as to block droplets or aerosols from entering the respiratory system of the user. The present invention can provide the non-enclosing device for blocking viruses and bacteria, the device using diamagnetism and wind power so as to push droplets and aerosols away from the face of a user such that the entering of the droplets or aerosols into the respiratory system and the outward spreading of the droplets of the user are blocked, and emitting visible sterilization light, which is harmless to the human body, so that viruses and bacteria contained in droplets and aerosols are killed.

## Description

### [Technical Field]

The present invention relates to a non-enclosing device for blocking viruses and bacteria, which does not block a mouth and a nose, and more particularly, to a non-enclosing device for blocking viruses and bacteria, capable of pushing droplets or aerosols including viruses or bacteria from a mouth and a nose by using a diamagnetic force and a wind force to block the droplets or the aerosols from being introduced into the mouth and the nose while the mouth and the nose are open, and capable of sterilizing the viruses and the bacteria through a visible-light sterilization lamp.

### [Background Art]

In general, a mask is an article that covers a nose and a mouth to prevent inhalation and scattering of pathogenic bacteria, dust, and the like for health and hygiene reasons.

In particular, masks are worn to prevent infection of diseases such as a cold, and mandatory wearing of masks has been recommended to prevent infection of COVID-19.

According to domestic and international disease prevention organizations including U.S. Centers for Disease Control and Prevention (CDC) and World Health Organization (WHO), relevant organizations, and experts, a main transmission route of COVID-19 is divided into three routes: first, airborne transmission by fine respiratory droplets and aerosol particles (airborne infection); second, spray transmission through droplets (droplet infection); and third, contact transmission by touching a nose, a mouth, and an eye with a contaminated hand (contact infection).

In detail, the airborne infection (airborne transmission) refers to infection of viruses in the form of aerosols including particles having a size less than 5 µm or a size of 0.001 to 1 µm to a respiratory organ when another person inhale air while the viruses float in the air. In particular, aerosols refer to fine water droplets that float in the air without being settled.

The droplet infection (droplet transmission) refers to infection caused in a case where small water droplets (droplets) such as saliva are mixed with viruses or bacteria to come out and enter a mouth and a nose of another person when an infected person coughs or sneezes. The droplets mean "water droplets that are spattered and scattered", a size of the droplet is 5 to 10 µm, and in general, when someone coughs once, about 3,000 droplets (respiratory droplets) are sprayed to fall within 2 m in a forward direction, and viruses may die. When the size of the droplet is less than or equal to 5 µm, the droplet is referred to as a droplet nucleus.

Therefore, in order to avoid the airborne infection and the droplet infection, it is recommended to stay away from the infected person by 2 m or more, and wear a mask.

However, since the mask encloses the mouth and the nose, the mask has to be removed when eating foods, so that a wearer of the mask may be exposed to droplets and aerosols of another person.

In addition, even when the mask is worn, a gap may be generated between a face and the mask through a facial movement and the like without achieving complete close contact or enclosing, and droplets and aerosols of another person may be introduced through the gap upon respiration.

In addition, infants, patients with brain lesions, patients with developmental disabilities, patients with respiratory disorders, and the like had difficulty in wearing masks.

### [Patent Documents]

(Patent document 1) Korean Unexamined Patent Publication No. 10-2019-0126674
(Patent document 2) Korean Unexamined Patent Publication No. 10-2022-0013818
(Patent document 3) Korean Unexamined Patent Publication No. 10-2020-0089569
(Patent document 4) Korean Patent Registration No. 10-1699789

### [Disclosure]

### [Technical Problem]

An object of the present invention, which has been devised to solve the problems described above, is to provide a non-enclosing device for blocking viruses and bacteria, capable of pushing droplets and aerosols away from a face of a user by using a diamagnetic force and a wind force to block the droplets and the aerosols from being introduced into a respiratory organ, and capable of blocking droplets of the user from spreading to an outside to prevent airborne infection and droplet infection of viruses and bacteria.

In addition, an object of the present invention is to provide a non-enclosing device for blocking viruses and bacteria, capable of radiating a visible sterilization light, which is harmless to a human body, to a face of a user to sterilize viruses and bacteria included in droplets and aerosols with a diamagnetic force and air, thereby preventing contact infection of the viruses and the bacteria.

In addition, an object of the present invention is to provide a non-enclosing device for blocking viruses and bacteria, in which the non-enclosing device may be detachably attached easily by hanging the non-enclosing device on a body of a user, such as a neck, a shoulder, or a head, and conversation and food consumption may be performed conveniently and safely through introduction and sterilization of viruses and bacteria by using at least one of a diamagnetic force, a wind force, and a visible sterilization light while a face of the user is open.

### [Technical Solution]

To achieve the objects described above, according to the present invention, there is provided a non-enclosing device for blocking viruses and bacteria, the non-enclosing device including: a main body detachably attached to a body of a user; and a blocking part provided on the main body to block droplets or aerosols from being introduced into a respiratory organ of the user.

In addition, the blocking part may include a magnetic part configured to generate a magnetic field to the respiratory organ of the user to generate a diamagnetic force so as to push the droplets or the aerosols away from the respiratory organ.

In addition, the blocking part may include an air supply part configured to supply air to the respiratory organ of the user to push the droplets or the aerosols away from the respiratory organ.

In addition, the magnetic part may be rotatably provided on the main body so that a magnetic field direction is adjusted.

In addition, the air supply part may be rotatably provided on the main body so that an air supply direction is adjusted.

In addition, the blocking part may further include an air supply part provided on the main body and configured to supply air to the respiratory organ of the user, and the magnetic part and the air supply part may be rotatably provided on the main body so that a magnetic field direction and an air supply direction are adjusted, respectively.

In addition, the main body may be formed in a band shape having one side that is opened so as to be hung around a neck or a shoulder, and may have both ends provided with blocking parts, respectively.

In addition, the blocking part may include a light radiation part configured to radiate a visible sterilization light to the respiratory organ of the user to sterilize viruses and bacteria included in the droplets or the aerosols.

In addition, the blocking part may further include a light radiation part configured to radiate a visible sterilization light to the respiratory organ of the user to sterilize viruses and bacteria included in the droplets or the aerosols, the non-enclosing device may further include a control part configured to control operation states of the magnetic part, the air supply part, and the light radiation part, and
the control part may be configured to control at least two of the magnetic part, the air supply part, and the light radiation part to operate simultaneously.

In addition, the magnetic part may protrude in a magnetic field direction, and may have one end rotatably provided on the main body.

In addition, the non-enclosing device may further include an angle adjustment part configured to connect the main body to the blocking part, and perform angle adjustment through forward-direction rotation or bending so that one side of the blocking part faces the respiratory organ of the user.

In addition, the non-enclosing device may further include: a detection sensor configured to detect a third party within a preset setting distance from the user wearing the main body; and a control part configured to control an operation of the blocking part according to a detection state of the third party.

In addition, the non-enclosing device may further include: a voice recognition part configured to recognize a voice of the user; and a control part configured to control an operation of the blocking part when a preset setting voice is recognized from the voice recognition part.

### [Advantageous Effects]

As reviewed above, an effect of the present invention is to provide a non-enclosing device for blocking viruses and bacteria, capable of pushing droplets and aerosols away from a face of a user by using a diamagnetic force and a wind force to block the droplets and the aerosols from being introduced into a respiratory organ, and capable of blocking droplets of the user from spreading to an outside to prevent airborne infection and droplet infection of viruses and bacteria.

In addition, an effect of the present invention is to provide a non-enclosing device for blocking viruses and bacteria, capable of radiating a visible sterilization light, which is harmless to a human body, to a face of a user to sterilize viruses and bacteria included in droplets and aerosols, thereby preventing contact infection of the viruses and the bacteria.

In addition, an effect of the present invention is to provide a non-enclosing device for blocking viruses and bacteria, in which the non-enclosing device can be detachably attached easily by hanging the non-enclosing device on a body of a user, such as a neck, a shoulder, or a head, and conversation and food consumption can be performed conveniently and safely through introduction and sterilization of viruses and bacteria by using at least one of a diamagnetic force, a wind force, and a visible sterilization light while a face of the user is open.

### [Description of Drawings]

FIG. 1 is a perspective view showing a non-enclosing device for blocking viruses and bacteria according to an exemplary embodiment of the present invention.
FIG. 2 is an exploded perspective view showing the non-enclosing device for blocking the viruses and the bacteria according to the exemplary embodiment of the present invention.
FIG. 3 is an enlarged view showing a movement of a blocking part of the non-enclosing device for blocking the viruses and the bacteria according to the exemplary embodiment of the present invention.
FIG. 4 is an enlarged view showing the blocking part of the non-enclosing device for blocking the viruses and the bacteria according to the exemplary embodiment of the present invention.
FIG. 5 is an enlarged view showing a movement of a magnetic part of the non-enclosing device for blocking the viruses and the bacteria according to the exemplary embodiment of the present invention.
FIG. 6 is an enlarged view showing an operation state of the blocking part of the non-enclosing device for blocking the viruses and the bacteria according to the exemplary embodiment of the present invention.
FIG. 7 is a view illustrating an example of using the non-enclosing device for blocking the viruses and the bacteria according to the exemplary embodiment of the present invention.
FIG. 8 is a view showing a configuration of the non-enclosing device for blocking the viruses and the bacteria according to the exemplary embodiment of the present invention.

### [Description of Reference Numerals]

10: Main body 11: Charging port
20: Blocking part 20a: Body
21: Magnetic part 22: Plunger
23: Coil 24: Air supply part
25: Shaft 26: Motor
27: Fan 27a: Outlet
28: Cover 29: Light radiation part
30: Angle adjustment part 31: Ball
32: Fitting groove 40: Power supply part
50: Control part 60: Manipulation part
61: Power button 62: Main function button
70: Display part

### [Best Mode]

Advantages and features of the present invention and methods for achieving the same will become clear with reference to the following detailed descriptions of embodiments taken in conjunction with the accompanying drawings. However, the present invention may be implemented in various different forms without being limited to the embodiments disclosed below, present embodiments are provided only so that the disclosure of the present invention will be complete, and will fully convey the scope of the invention to a person having ordinary skill in the art to which the present invention pertains, and the present invention will be defined only by the scope of the claims. Like reference numerals refer to like elements throughout the present disclosure.

Hereinafter, the present invention will be described with reference to the drawings for describing a non-enclosing device for blocking viruses and bacteria through embodiments of the present invention.

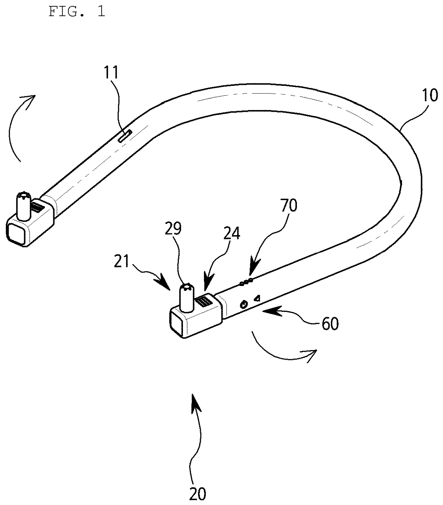

Referring to FIG. 1, according to the present invention, the non-enclosing device for blocking the viruses and the bacteria may include a main body 10 and a blocking part 20.

First, the main body 10 may be detachably attached to a body of a user.

For example, the main body 10 may be formed in a 'C' shape having one side that is opened so as to be fitted around a neck of the user.

In this case, the main body 10 may be fitted around the neck to allow both ends to be located at a collarbone region of the user, may fixedly surround the neck, or may be fixedly seated on a shoulder. In addition, a length adjustment part configured to adjust a length of each of both end portions of the main body 10 so that a region where the main body 10 is seated on the neck or the shoulder may be adjusted according to a body structure or a body size of the user may be provided.

In addition, a middle portion of the main body 10 may have elasticity so that the both ends may return to their original states after being spread.

The main body 10 of the non-enclosing device for blocking the viruses and the bacteria according to the present invention may be formed in a band shape having one side that is opened so as to be hung around a neck or a shoulder, and may have both ends provided with blocking parts 20, respectively.

For example, the main body 10 may be formed as a 'C'-shaped neckband that may be worn on the neck or the shoulder. As another example, the main body 10 may be formed in an 'O' shape so as to be placed on a head, or may be formed in a pincers or ring shape so as to be fixed to clothes of the user or the like, so that the main body 10 may be formed in various shapes without being limited thereto.

A power supply part 40 configured to supply a power for driving the blocking part 20 that will be described below may be provided inside the main body 10. In this case, the power supply part 40 may be provided inside the blocking part 20 that will be described below, and may be configured as a rechargeable battery. In addition, a charging port 11 electrically connected to the power supply part 40 so that the power supply part 40 may be charged with an external power source may be formed on an outer side of the main body 10.

In this case, the charging port 11 may be formed in micro 5-pin, lightning 8-pin, USB-C type, or the like, but is not limited thereto.

The blocking part 20 may be provided on the main body 10 to block droplets or aerosols from being introduced into a respiratory organ of the user. In other words, the blocking part 20 may push the droplets or the aerosols away from the respiratory organ of the user, or sterilize the droplets or the aerosols.

In addition, the blocking part 20 may be rotatably provided at each of the both end portions of the main body 10 formed in a 'C' shape.

In other words, since the size and the structure of the body vary for each user, while the main body 10 is worn on the neck or the shoulder, the blocking part 20 may be adjusted to face the respiratory organ of the user so as to push or sterilize the droplets or the aerosols around the respiratory organ of the user.

In this case, the blocking part 20 may include a magnetic part 21, an air supply part 24, and a light radiation part 29, which will be described below, provided on a body 20a.

In this case, according to the present invention, the non-enclosing device for blocking the viruses and the bacteria may further include an angle adjustment part 30.

The angle adjustment part 30 may connect the main body 10 to the blocking part 20, and perform angle adjustment through forward, rearward, left, and right-direction rotation or bending so that one side of the blocking part 20 may face the respiratory organ of the user. The magnetic part 21 and the air supply part 24 of the blocking part 20, which will be described below, may be rotated or bent by the angle adjustment part 30 so that angles of a magnetic field direction and an air supply direction may be adjusted.

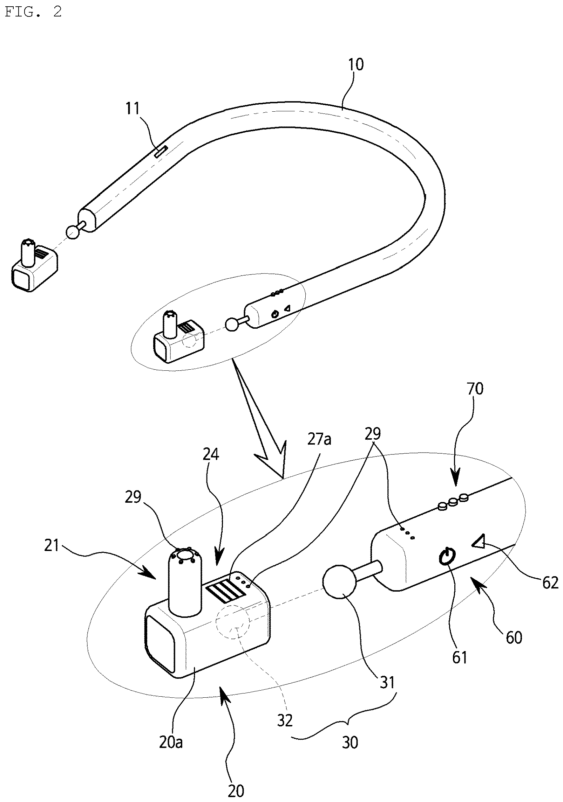

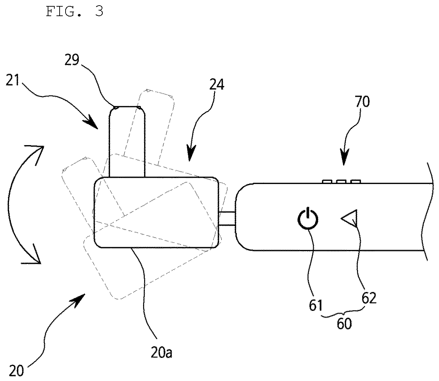

For example, the angle adjustment part 30 may connect the main body 10 to the blocking part 20 in a ball-joint fastening structure. Referring to FIGS. 2 and 3, a ball 31 may protrude from the main body 10, and a fitting groove 32 in which the ball 31 is fitted and forcedly rotated may be formed on an opposite side of the blocking part 20. In other words, the blocking part 20 may be freely moved to change a direction and an angle that one side of the blocking part 20 faces, and may be coupled to be forcedly rotated to firmly maintain an adjusted direction and an adjusted angle against external vibrations or impacts.

In addition, the angle adjustment part 30 may include a bending member formed of a material such as a metal capable of maintaining a bent state when bent. In addition, the angle adjustment part 30 may include a joint part configured as a plurality of joints connected to each other so as to be bent, or may have a hinge structure and the like.

As described above, the angle adjustment part 30 may have various structures, which enable adjustment of the direction and the angle that the one side of the blocking part 20 faces and fixation in an adjusted state.

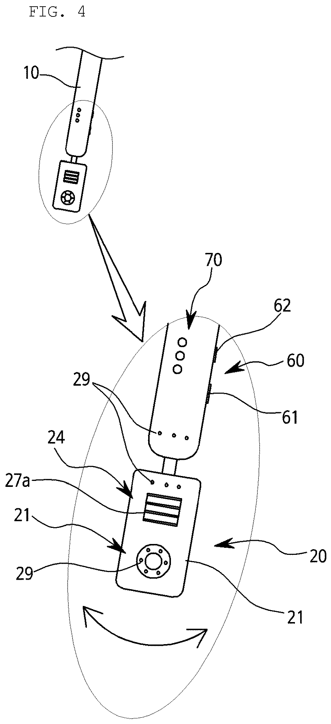

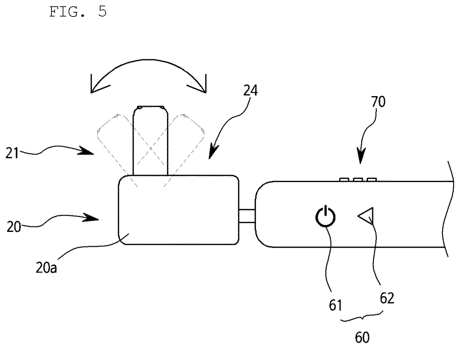

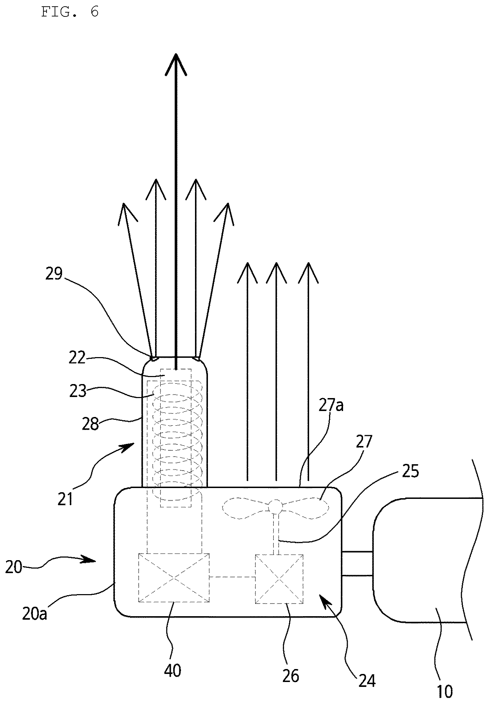

Meanwhile, referring to FIGS. 4 to 7, the blocking part 20 may include at least one of the magnetic part 21, the air supply part 24, and the light radiation part 29 so as to block the droplets or the aerosols from being introduced into the respiratory organ of the user.

First, the magnetic part 21 may generate a magnetic field to the respiratory organ of the user to generate a diamagnetic force in the droplets or the aerosols.

In this case, since the droplets or the aerosols are formed of water droplets, a diamagnetic force may be generated against the magnetic field.

In this case, diamagnetism refers to a phenomenon in which a direction of magnetization is formed opposite to a direction of a magnetic field when the magnetic field is applied to a material. In this case, a representative diamagnetic body may include gas, water, and the like, excluding a metal and oxygen.

In other words, since the droplets or the aerosols are formed of water droplets to float in the air, the droplets or the aerosols may be pushed in a direction opposite to the direction of the magnetic field when the magnetic field is applied from the magnetic part 21.

In addition, the magnetic part 21 may be rotatably provided on the main body 10 so that a magnetic field direction may be adjusted. In this case, the magnetic part 21 may be forcedly rotated with respect to the main body 10 so that the magnetic field direction may be adjusted, and the magnetic part 21 may be fixed in an adjusted state.

In other words, the magnetic part 21 may be configured such that the magnetic field direction is set to the respiratory organ of the user when an angle is adjusted to allow the magnetic field direction to face the respiratory organ of the user, so that the droplets or the aerosols around the respiratory organ of the user may be pushed away from the respiratory organ of the user by the diamagnetic force, and the user may be prevented from being infected with viruses and bacteria.

Meanwhile, the magnetic part 21 may protrude in the magnetic field direction. In other words, the magnetic part 21 may protrude from one side of the body 20a of the blocking part 20, and may be rotatably provided.

In this case, the magnetic part 21 may be an electromagnet or a permanent magnet including a plunger and a coil 23 wound around the plunger, but is not limited thereto.

In addition, the magnetic part 21 may include the coil 23 electrically connected to the power supply part 40. Accordingly, the magnetic field may be applied to the droplets or the aerosols depending on whether the power supply part 40 applies a power to the magnetic part 21.

In addition, the magnetic part 21 may have a cover 28 that surrounds the plunger and the coil 23.

Meanwhile, the blocking part 20 may further include the air supply part 24.

The air supply part 24 may supply air to the respiratory organ of the user to push the droplets or the aerosols away from the respiratory organ.

In this case, the air supply part 24 may include a motor 26 configured to receive the power from the power supply part 40 to rotate a shaft 25, and a fan 27 coupled to the shaft 25. In other words, the fan 27 may discharge the air to one side of the blocking part 20. In this case, although the motor 26 has been shown as being provided inside the blocking part 20, the motor 26 may be provided inside the main body 10. In addition, an outlet 27a may be formed on one side of the body 20a, and an inlet may be formed on an opposite side of the body 20a, so that external air may be introduced into and discharged from an inside of the blocking part 20 by rotation of the fan 27.

In addition, the air supply part 24 may be rotatably provided on the main body 10. Accordingly, an air supply direction may be adjusted.

In other words, the air supply direction may be adjusted to face the respiratory organ of the user according to the body structure or the body size of the user.

In this case, although the air supply part 24 and the magnetic part 21 have been shown in the drawing of the non-enclosing device for blocking the viruses and the bacteria according to the present invention as being provided together on the body 20a of the blocking part 20, embodiments are not limited thereto, and each of a first blocking part including the magnetic part 21 or the air supply part 24 and a second blocking part including the air supply part 24 or the magnetic part 21 may be provided on the main body 10.

In this case, when the configuration is divided into the first blocking part and the second blocking part, the first blocking part and the second blocking part may be rotatably provided on the main body 10 so that the magnetic field direction and the air supply direction may be adjusted, respectively. In addition, the first blocking part may be rotatably provided on the main body 10, and the second blocking part may be rotatably provided on the first blocking part.

In addition, since the magnetic part 21 and the air supply part 24 push the droplets or the aerosols around the respiratory organ of the user outward of the respiratory organ of the user, the user may adjust the magnetic field direction of the magnetic part 21 and the air supply direction of the air supply part 24 from a rear side of the user to a forward direction, a diagonal direction, or the like.

In addition, the magnetic field of the magnetic part 21 may serve to push aerosols having small sizes, and the air of the air supply part 24 may serve to push aerosols and droplets having larger sizes than the aerosols, but embodiments are not limited thereto.

Meanwhile, the blocking part 20 may further include a light radiation part 29.

The light radiation part 29 may radiate a visible sterilization light to the respiratory organ of the user to sterilize viruses and bacteria included in the droplets or the aerosols.

In this case, a visible light refers to a light having a wavelength range perceived by an eye, which is an electromagnetic wave having a wavelength of approximately 380 to 780 nm (nanometers). A wavelength of 780 nm or more corresponds to an infrared light and a heat ray used in a radio, and a wavelength of 380 nm or less corresponds to an ultraviolet light, an x-ray, and the like.

In this case, a visible light wavelength of 405 nm may react with oxygen to generate active oxygen that is harmless to a human body. In addition, the visible light wavelength of 405 nm may sterilize harmful bacteria such as Escherichia coli, Staphylococcus aureus, pneumococcus, and methicillin-resistant Staphylococcus aureus (MRSA) by 99.9%.

The sterilization principle of the visible light wavelength of 405 nm is that the visible light wavelength of 405 nm reacts with porphyrin of bacteria or the like to generate active oxygen, thereby destroying the bacteria and the viruses.

In other words, the visible sterilization light may include a visible light wavelength of 405 nm, which is not a wavelength harmful to the human body like the ultraviolet light, so that the visible sterilization light may be directly radiated to skin of the human body. Accordingly, the light radiation part 29 may sterilize the viruses and the bacteria included in the droplets or the aerosols floating around the respiratory organ of the user.

In addition, the light radiation part 29 may be provided at the outlet 27a or the inlet of the air supply part 24 so as to sterilize the external air that is introduced by the fan 27 of the air supply part 24 described above, or sterilize the air that is discharged.

In addition, the light radiation part 29 may be provided on one side of the blocking part 20, and the blocking part 20 may adjust a sterilization light radiation direction of the light radiation part 29 through rotation, bending, or the like.

In this case, although the non-enclosing device for blocking the viruses and the bacteria according to the present invention has been shown in the drawings as being configured such that the air supply part 24, the magnetic part 21, and the light radiation part 29 are provided together on the body 20a of the blocking part 20, embodiments are not limited thereto, each of a first blocking part including the magnetic part 21 or the air supply part 24, a second blocking part including the air supply part 24 or the magnetic part 21, and a third blocking part including the light radiation part 29 may be provided on the main body 10, and the light radiation part 29 may be provided in the first blocking part or the second blocking part, and may be provided on the main body 10, so that embodiments are not limited thereto.

In addition, since it may be efficient to allow the light radiation part 29 to sterilize a periphery of the user, a plurality of light radiation parts 29 may be provided in a longitudinal direction of the main body 10, and a plurality of light radiation parts may be provided on the blocking part 20.

*In addition, when the magnetic part 21, the air supply part 24, and the light radiation part 29 are provided integrally with each other, the magnetic field direction of the magnetic part 21, the air supply direction of the air supply part 24, and a sterilization light radiation direction of the light radiation part 29 may be set so as not to interfere with each other.

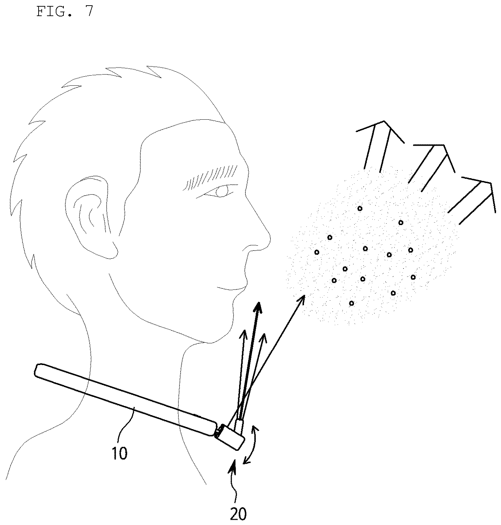

Therefore, as shown in FIG. 7, the user may adjust the angle of the blocking part 20 while wearing the main body 10 on the neck and the shoulder. In this case, since the magnetic part 21 and the air supply part 24 may separately perform angle adjustment, the magnetic field direction and the air supply direction may be adjusted so as to be different from each other.

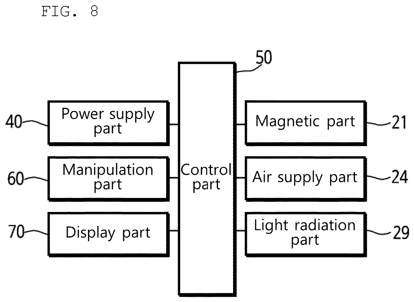

Meanwhile, referring to FIG. 8, according to the present invention, the non-enclosing device for blocking the viruses and the bacteria may further include a control part 50 and a manipulation part 60.

The control part 50 may control operation states of the magnetic part 21, the air supply part 24, and the light radiation part 29.

In addition, the control part 50 may control at least two of the magnetic part 21, the air supply part 24, and the light radiation part 29 to operate simultaneously.

The manipulation part 60 may be provided on the main body 10 or the blocking part 20 to input operation signals of the magnetic part 21, the air supply part 24, and the light radiation part 29.

For example, the manipulation part 60 may include a power button 61, a main function button 62, and an adjustment button.

The power button 61 may input an operation signal capable of turning the power of the power supply part 40 on/off. In this case, for the on/off of the power, the operation signal may be input when the power button 61 is pressed for a long time.

In addition, the main function button 62 may input an operation signal for operating one of the magnetic part 21, the air supply part 24, and the light radiation part 29, which are preset. In this case, when the main function button 62 is pressed once, the operation signal of the magnetic part 21 may be input, when the main function button 62 is pressed secondly or pressed twice consecutively, the operation signal of the air supply part 24 may be input, and when the main function button 62 is pressed thirdly or pressed three times consecutively, the operation signal of the light radiation part 29 may be input.

In addition, while at least one of the magnetic part 21, the air supply part 24, and the light radiation part 29 is operating with the main function button 62, when the power button 61 is pressed briefly, an operation signal for operating one more component among the magnetic part 21, the air supply part 24, and the light radiation part 29, which are not operating, may be input.

The adjustment button may input an operation signal capable of individually adjusting or collectively adjusting intensities of the magnetic part 21, the air supply part 24, and the light radiation part 29.

For example, the adjustment button may adjust an intensity of the magnetic field of the magnetic part 21 through adjustment of an intensity of the applied power, adjust a rotation speed of the fan 27 of the air supply part 24, and adjust an illuminance of the light radiation part 29.

In addition, the control part 50 may individually operate or simultaneously operate the magnetic part 21, the air supply part 24, and the light radiation part 29 each time when an operation signal is input by the manipulation part 60 according to preset settings.

In this case, since a scheme of inputting the operation signals of the magnetic part 21, the air supply part 24, and the light radiation part 29 by using the power button 61 and the main function button 62 may be variously set, embodiments are not limited thereto.

In addition, according to the present invention, the non-enclosing device for blocking the viruses and the bacteria may further include a display part 70 configured to check an on/off state of the power supply part 40, the operation states of the magnetic part 21, the air supply part 24, and the light radiation part 29, a remaining battery level, an intensity state, and the like. In this case, the display part 70 may include a lamp or a display portion, but is not limited thereto.

In addition, according to the present invention, the non-enclosing device for blocking the viruses and the bacteria may further include a checking part configured to allow the user to check input of the operation signal by the manipulation part 60. In this case, the checking part may include a vibration motor configured to generate vibrations, a speaker configured to output a sound, or the like, but is not limited thereto.

Meanwhile, according to the present invention, the non-enclosing device for blocking the viruses and the bacteria may further include a detection sensor and a voice recognition part.

The detection sensor may detect a third party within a preset setting distance from the user wearing the main body 10. In this case, the detection sensor may include an infrared sensor and the like, but is not limited thereto.

In addition, the control part 50 may control an operation of the blocking part according to a detection state of the third party.

In other words, the control part 50 may control the blocking part 20 with a preset operation signal according to a setting distance from the third party.

For example, in a case where the user wearing the non-enclosing device for blocking the viruses and the bacteria is talking or eating with the third party, the control part 50 may input an operation signal for operating all of the magnetic part 21, the air supply part 24, and the light radiation part 29 when a distance at which the third party is detected is within 50 cm, and may input an operation signal for operating only the magnetic part 21 when the distance is outside 50 cm.

As another example, the setting distance may be set as a first distance and a second distance that is further than the first distance. In this case, the control part 50 may operate all of the magnetic part 21, the air supply part 24, and the light radiation part 29 when a distance between the user and the third party is within the first distance, operate the magnetic part 21 and the air supply part 24 when the distance is within the first distance and the second distance, and operate only the magnetic part 21 when the distance is further than the second distance. Since the above configuration is provided for illustrative purposes, embodiments are not limited thereto, and control of the blocking part 20 may vary according to settings.

The voice recognition part may recognize a voice of the user.

In this case, the control part 50 may control an operation of the blocking part 20 when a preset setting voice is recognized from the voice recognition part. In addition, a preset operation signal may be set for the setting voice.

In other words, when the user speaks the preset setting voice, the voice recognition part may recognize the setting voice, and the blocking part 20 may operate with the preset operation signal according to the setting voice.

For example, the setting voice may include "operate the magnetic part", and when the voice "operate the magnetic part" is recognized, the control part 50 may operate the magnetic part 21. Since the above configuration is provided for illustrative purposes, embodiments are not limited thereto, and the blocking part 20 may be variously controlled with various setting voices.

As described above, according to the non-enclosing device for blocking the viruses and the bacteria of the present invention, the operation signals of the magnetic part 21, the air supply part 24, and the light radiation part 29 may be input in various schemes, so that embodiments are not limited thereto.

It will be understood by a person having ordinary skill in the art to which the present invention pertains that the present invention may be implemented in other specific forms without changing the technical idea or essential characteristics thereof. Therefore, the embodiments described above are illustrative in all respects and should not be construed as limiting. The scope of the present invention is to be indicated by the appended claims rather than the detailed description, and interpreted as encompassing all changes or modified forms derived from the meaning and scope of the claims and equivalent concepts thereof. In addition, an operation order of components described in the above-described process does not necessarily have to be a chronological order, and in a case where the gist of the present invention is satisfied even when an execution order of the components and steps is changed, such a process may fall within the scope of the present invention.

## Claims

1. A non-enclosing device for blocking viruses and bacteria, the non-enclosing device comprising:
a main body detachably attached to a body of a user; and
a blocking part provided on the main body to block droplets or aerosols from being introduced into a respiratory organ of the user,
wherein the blocking part includes a magnetic part configured to generate a magnetic field to the respiratory organ of the user to generate a diamagnetic force so as to push the droplets or the aerosols away from the respiratory organ.

2. The non-enclosing device of claim 1, wherein the blocking part includes an air supply part configured to supply air to the respiratory organ of the user to push the droplets or the aerosols away from the respiratory organ.

3. The non-enclosing device of claim 1, wherein the magnetic part is rotatably provided on the main body so that a magnetic field direction is adjusted.

4. The non-enclosing device of claim 2, wherein the air supply part is rotatably provided on the main body so that an air supply direction is adjusted.

5. The non-enclosing device of claim 1, wherein the blocking part further includes an air supply part provided on the main body and configured to supply air to the respiratory organ of the user, and
the magnetic part and the air supply part are rotatably provided on the main body so that a magnetic field direction and an air supply direction are adjusted, respectively.

6. The non-enclosing device of claim 1, wherein the blocking part includes a light radiation part configured to radiate a visible sterilization light to the respiratory organ of the user to sterilize viruses and bacteria included in the droplets or the aerosols.

7. The non-enclosing device of claim 5, wherein the blocking part further includes a light radiation part configured to radiate a visible sterilization light to the respiratory organ of the user to sterilize viruses and bacteria included in the droplets or the aerosols,
the non-enclosing device further comprises a control part configured to control operation states of the magnetic part, the air supply part, and the light radiation part, and
the control part is configured to control at least two of the magnetic part, the air supply part, and the light radiation part to operate simultaneously.

8. The non-enclosing device of claim 1, wherein the magnetic part protrudes in a magnetic field direction, and has one end rotatably provided on the main body.

9. The non-enclosing device of claim 1, further comprising an angle adjustment part configured to connect the main body to the blocking part, and perform angle adjustment through forward-direction rotation or bending so that one side of the blocking part faces the respiratory organ of the user.

10. The non-enclosing device of claim 1, further comprising:
a detection sensor configured to detect a third party within a preset setting distance from the user wearing the main body; and
a control part configured to control an operation of the blocking part according to a detection state of the third party.

11. The non-enclosing device of claim 1, further comprising:
a voice recognition part configured to recognize a voice of the user; and
a control part configured to control an operation of the blocking part when a preset setting voice is recognized from the voice recognition part.
